# EUROPEAN PATENT SPECIFICATION

(11) **EP 4 306 155 B1**
(45) Date of publication and mention of the grant of the patent: **21.01.2026**
(21) Application number: 22766390.3
(22) Date of filing: 11.03.2022
(51) Int. Cl.: A61M 16/00, A61M 16/06

(54) **BREATHING CONTROL ASSEMBLY AND VENTILATION THERAPY DEVICE**
ATEMSTEUERUNGSANORDNUNG UND BEATMUNGSTHERAPIEVORRICHTUNG
ENSEMBLE DE CONTRÔLE DE RESPIRATION ET DISPOSITIF DE THÉRAPIE DE VENTILATION

(30) Priority: 12.03.2021 CN 202110272357
(43) Date of publication of application: 17.01.2024
(73) Proprietor: BMC (Tianjin) Medical Co., Ltd., Tianjin 301700 (CN)
(72) Inventor: HE, Long, Tianjin 301700 (CN); ZHOU, Mingzhao, Tianjin 301700 (CN); ZHUANG, Zhi, Tianjin 301700 (CN)
(74) Representative: Grünecker Patent- und Rechtsanwälte PartG mbB
(86) International application number: PCT/CN2022/080396
(87) International publication number: WO 2022/188868

(56) References cited:
- WO-A1-2014/013411
- CN-A- 102 380 147
- CN-A- 105 686 847
- CN-A- 110 101 530
- CN-A- 113 101 477
- JP-A- 2003 079 732
- US-A1- 2013 178 716
- US-A1- 2016 287 145
- US-A1- 2018 242 900

## Description

### CROSS-REFERENCE TO RELATED APPLICATION

The present application claims the priority Chinese patent application filed on March 12th, 2021 before the China National Intellectual Property Administration with the application number of 202110272357.5, and the title of "RESPIRATORY CONTROL ASSEMBLY AND VENTILATION THERAPY APPARRATUS".

### TECHNICAL FIELD

The present disclosure relates to the technical field of medical devices and more particularly, to a respiratory control assembly and a ventilation therapy apparatus.

### BACKGROUND

A ventilation therapy apparatus generally includes an airflow generator, a gas transmission pipe and a user interface device. The user interface device sends positive pressure airflow generated by the airflow generator into a user's airway, to realize positive pressure ventilation therapy. If the user swallows or yawns during use, the eustachian tube of the user is opened. At this time, since the ventilation therapy apparatus is still transporting positive pressure airflow to the user, and the eustachian tube is unable to be closed by itself in a positive pressure environment, a potential risk that the user may have problems for example "ear perforation" and the like exists, and in severe cases, it even leads to users' hearing loss. Consequently, a ventilation therapy apparatus in related art has a certain potential safety hazard.

WO 2024/013411 A1 discloses a system for providing positive pressure respiratory therapy to a subject. The system comprises a swallow detection module, a target module and a control module. The swallow detection module is used for detecting swallowing action of a subject so as to reduce a target pressure temporarily so as to reduce aerophagia and improve compliance.

US 2018/242900 A1 discloses a swallowing movement monitoring sensor as an alternative to an endoscopic swallowing examination or videofluoroscopic examination of swallowing for assessing a swallowing function. This is done by a first outside shape variation sensor attached to a laryngeal portion of the subject and a microphone attached in an ear of the subject. The microphone detects swallowing sound produced in the ear in association with swallowing movement.

### SUMMARY

The invention is defined in the appended independent claim 1. Preferred embodiments are matter of the dependent claims.

The technical problem to be solved by the embodiment of the present application is to provide a respiratory control assembly and a ventilation therapy apparatus, which are capable to stop transporting gas to a user when it is detected that the eustachian tube of the user is open, and re-transport the gas when it is detected that the eustachian tube of the user is closed, to eliminate health hazards when a user uses the respiratory control assembly.

In order to solve the above-mentioned problem, an embodiment of a first aspect of the present application provides a respiratory control assembly, including:
an acquisition module, a control module and a gas transmission module, wherein,
the acquisition module is connected with the control module in communication, and is used for collecting a gas parameter of an eustachian tube of a user and sending the gas parameter to the control module; and
the control module is connected with the gas transmission module in communication, and the control module is used for determining opening and closing states of the eustachian tube according to the gas parameter, and configuring a gas transmission state of the gas transmission module according to the opening and closing states.

Optionally, the acquisition module includes an air pressure detector and an in-ear structure for accommodating the air pressure detector, wherein the gas parameter is an air pressure of an external auditory canal connected with the eustachian tube, and the air pressure detector is used for collecting the air pressure of the external auditory canal; and
the control module is further configured for, when it is detected that the air pressure of the external auditory canal is greater than an initial air pressure, determining that the eustachian tube is open ,and controlling the gas transmission module to stop transporting gas, and when it is detected that the air pressure of the external auditory canal drops to less than or equal to the initial air pressure, determining that the eustachian tube is closed, and controlling the gas transmission module to continue transporting the gas.

Optionally, the acquisition module further includes: an ear hanging structure connected with the in-ear structure, and the ear hanging structure is used for being hung on an auricle of the user.

Optionally, the acquisition module includes an acoustic wave detector and a patch structure for accommodating the acoustic wave detector, wherein the acoustic wave detector is used for collecting acoustic wave information of breathing airflow; and
the control module is used for, when it is detected that matching degree between a waveform of the acoustic wave information and a preset waveform is less than a preset matching threshold, determining that the eustachian tube is open, and controlling the gas transmission module to stop transporting the gas, and when it is detected that the matching degree is greater than or equal to the preset matching threshold, determining that the eustachian tube is closed, and controlling the gas transmission module to continue transporting the gas.

Optionally, the respiratory control assembly further includes:
a prompting module connected with the control module in communication, and the prompting module is used for sending prompting information when it is determined by the control module that the eustachian tube is under a working condition of being open.

Optionally, the gas transmission module includes an airflow generator and a controlled device which are connected in communication, and a gas transmission pipe which is connected with the airflow generator; the controlled device and the control module are connected in communication, and the controlled device receives a control parameter of the control module, to control the airflow generator to transport gas through the gas transmission pipe or stop transporting gas according to the control parameter.

Optionally, the controlled device is a switch controller of the airflow generator, one end of the gas transmission pipe is communicated with the airflow generator, and the other end of the gas transmission pipe is used for transporting the gas to the user; the control parameter is used for controlling the switch controller to turn off the airflow generator when the eustachian tube is under the working condition of being open, and the control parameter is used for controlling the switch controller to turn on when the eustachian tube is under the working condition of being closed.

Optionally, the controlled device is a control valve, the control valve is used for controlling the gas transmission pipe to be communicated or to be cut-off, and the control parameter is used for controlling the control valve to be closed when the eustachian tube is under the working condition of being open, and the control parameter is used to control the control valve to be open when the eustachian tube is under the working condition of being closed.

An embodiment of a second aspect of the present application provides a ventilation therapy apparatus, including:
a breathing mask;
the respiratory control assembly according to any one of the embodiments of the first aspect of the present application, and the respiratory control assembly includes a gas transmission pipe and an airflow generator, wherein one end of the gas transmission pipe is connected to the breathing mask, and the other end of the gas transmission pipe is connected to the airflow generator.

Optionally, the respiratory control assembly includes a controlled device; under the condition that the controlled device is a control valve, the control valve is disposed on the gas transmission pipe, and/or the control valve is disposed at a joint of the gas transmission pipe and the breathing mask, and/or the control valve is disposed at a joint of the gas transmission pipe and the airflow generator.

The respiratory control assembly defined in the embodiment of the present application includes an acquisition module, a control module and a gas transmission module which are connected in sequence in communication, to form a servo control system for controlling the gas transmission state of the gas transmission module based on the gas parameter of a user's eustachian tube collected by the acquisition module. Particularly, the acquisition module transmits the collected gas parameter of the user's eustachian tube to the control module. The control module is capable to determine whether the eustachian tube is under an opening state or a closing state according to whether the gas parameter changes. If the eustachian tube is under the opening state, the control module generates a control instruction to control the gas transmission module to stop transporting gas, to eliminate the positive pressure in the eustachian tube, and then it makes the eustachian tube close by itself. If the eustachian tube is under the closing state, the control module generates a control instruction to control the gas transmission module to continue transporting gas, so that the respiratory control assembly is capable to continuously transport gas to the user for treatment.

By providing the respiratory control assembly with the acquisition module, when a user is treated, the respiratory control assembly is capable to improve the reliability of operation for a user with autonomous ability, so that the treatment effect is effectively improved, and for a user without autonomous ability, the respiratory control assembly is capable to prevent the user from generating the risks of problems for example ear perforation and hearing loss and the like during the treatment process, to improve the safety of operation.

### BRIEF DESCRIPTION OF THE DRAWINGS

FIG. 1 is a schematic diagram of the ventilation therapy apparatus in the related art;
FIG. 2 is a schematic diagram of the closing state of the eustachian tube in the human body;
FIG. 3 is a schematic diagram of the opening state of the eustachian tube in the human body;
FIG. 4 is a schematic block diagram of an embodiment of the respiratory control assembly of the present application;
FIG. 5 is a schematic diagram of another embodiment of the respiratory control assembly of the present application;
FIG. 6 is a schematic diagram when the eustachian tube is open in an embodiment of the respiratory control assembly of the present application;
FIG. 7 is a schematic diagram when the eustachian tube is closed in an embodiment of the respiratory control assembly of the present application; and
FIG. 8 is a structural block diagram of an embodiment of the ventilation therapy apparatus of the present application.

### DETAILED DESCRIPTION

In order to make the above-mentioned purposes, features and advantages of the present application more apparent and easier to understand, the present application will be further described in detail with the drawings and particular embodiments.

In the present application, orientation words for example "top or bottom" are generally used for referring to the orientation shown in the drawings, unless otherwise stated. "Inside or outside" refers to the inside and outside relative to the outline of every component itself.

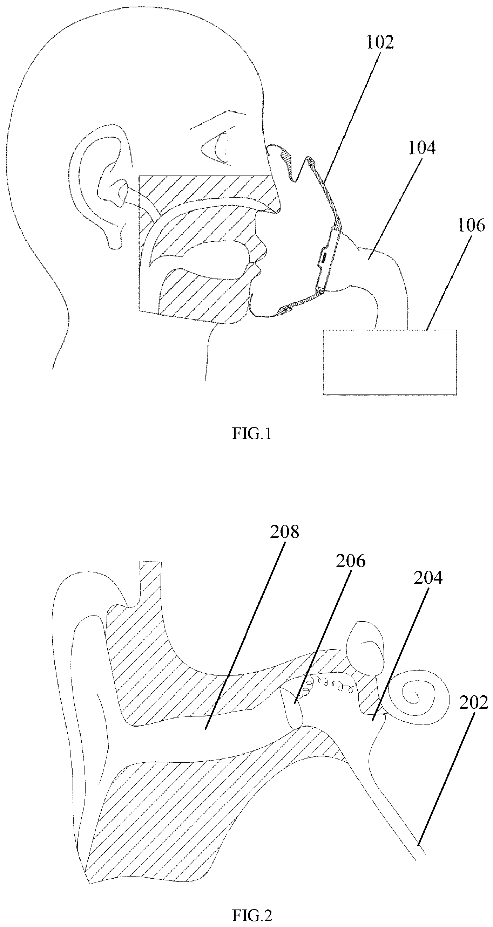

As shown in FIG. 1, a ventilation therapy apparatus in the related art includes an interface device 102 for covering a user's nose and mouth, an airflow generator 106, and a gas transmission pipe 104 connecting the interface device 102 and the airflow generator 106. During the treatment, the interface device 102 covers the nose and mouth and/or mouth of a user, and the airflow generated by the airflow generator 106 enters a nasal cavity tube and/or an oral cavity tube of the user through the gas transmission pipe 104 and the interface device 102. When the user swallows or yawns, the eustachian tube (also known as Euclidean tube, which is an important channel to communicate with the tympanum and nasopharynx, is merely open when swallowing or yawning, to balance the air pressure of the middle ear and the outer ear, which is beneficial to the normal vibration of the eardrum) of the user is open. At this time, the ventilation therapy apparatus still transports positive pressure airflow to the user, but the eustachian tube is uncapable to be closed by itself under a positive pressure environment.

When under the working condition that the eustachian tube is open, a user who has the autonomous processing capability is required to automatically interrupt the treatment, and the treatment is continued after the eustachian tube is closed, the operation is troublesome, and the treatment effect is affected. For a user does not have autonomous treatment ability, when the eustachian tube is open, the treatment is uncapable to be interrupted in time, and the eardrum communicated with the eustachian tube will continue to be blown by the airflow of the ventilator, so that a potential risk that the user may have problems for example "ear perforation" and the like exists, and in severe cases, it even leads to users' hearing loss. Consequently, a ventilation therapy apparatus in the related art has a certain potential safety hazard.

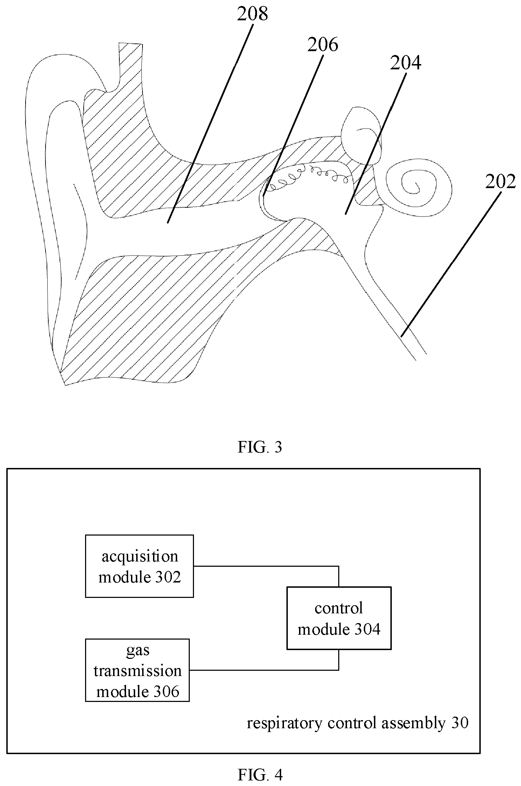

FIG. 2 shows a schematic diagram of the eustachian tube under a closing state. The eustachian tube 202 is a gas transmission pipe leading from the tympanum to the nasopharynx behind the nasal cavity in the human body, with a length of about 3.5 cm to 4.0 cm. One third of the eustachian tube 202 that close to the tympanum is composed of hard bones, and two thirds of the eustachian tube 202 that close to the nasopharynx is composed of cartilage. The anterior wall of the tympanum has a tympanic orifice of the eustachian tube 202, and the opening connected with the nasopharynx is a pharyngeal orifice of the eustachian tube 202. Among them, the tympanic orifice always remains unobstructed, and the pharyngeal orifice is similar to a one-way valve. As shown in FIG. 2, the pharyngeal orifice is usually under the closing state, and merely liquid or gas in the middle ear is allowed to overflow, and nasopharyngeal secretions and bacteria are not allowed to enter the tympanum, so that the ear-drum 206 and a round window membrane are in the best vibration position, and normal hearing is obtained. The pharyngeal orifice of the eustachian tube 202 is open due to the contraction of the pharyngeal muscle merely when the mouth is open, singing, chewing, yawning or swallowing. As shown in FIG. 3, at this point, external air is capable to enter the tympanum, so that the pressure inside and outside the tympanum is balanced, and normal sound transmission of the middle ear is ensured.

During the treatment, when a user swallows or yawns, the pharyngeal orifice of the eustachian tube 202 is open, and the airflow from the respiratory control assembly enters the middle ear canal 204 through the eustachian tube 202. The air pressure in the middle ear is greater than the air pressure in the outer ear, and the ear-drum 206 bulges toward the external ear canal 208. At this time, the ear-drum 206 is uncapable to vibrate normally, so that the hearing of the user is affected. If the air pressure in the middle ear continues to increase to be greater than the bearing capacity of the ear-drum 206, the ear-drum 206 will rupture, causing ear perforation, and causing permanent hearing loss of a user.

Based on the potential safety hazard existing in the use process of the existing respiratory control assembly 30, referring to FIG. 4, a structural diagram of the respiratory control assembly 30 of the present application is shown, and the respiratory control assembly 30 includes: an acquisition module 302, a control module 304, and a gas transmission module 306.

Among them, the acquisition module 302 is connected with the control module 304 in communication, and is used for collecting a gas parameter of an eustachian tube 202 of a user and sending the gas parameter to the control module 304.

Particularly, since the eustachian tube 202 is in the human body, the acquisition module 302 collects the gas parameter of the eustachian tube 202, the particular implementation mode is to collect the gas parameter transported through the eustachian tube 202 or the gas parameter in the external ear canal 208 separated from the eustachian tube 202 through the eardrum. The gas parameter may be an air pressure parameter, a breathing frequency parameter of breathing gas in the eustachian tube 202 or a breathing acoustic wave parameter of the breathing gas, and so on. The acquisition module 302 is particularly a sensor that collects the above-mentioned parameters.

The control module 304 is connected with the gas transmission module 306 in communication, and the control module 304 is used for determining opening and closing states of the eustachian tube 202 according to the gas parameter, and configuring a gas transmission state of the gas transmission module 306 according to the opening and closing states.

The control module 304 is specifically a control chip, the control module 304 determines the opening and closing states of the eustachian tube 202 based on the acquired gas parameters. Specifically, for example, when the detected gas parameter is changing, it can determine whether the changing amplitude exceeds a preset amplitude range, and it determines whether to generate the control parameter for adjusting the gas transmission module 306 based on the detection results.

The gas transmission module 306 is used to transport gas to a user and assist the user to breathe. The gas transmission module 306 is connected with the control module 304 in communication to be controlled by the control module 304 to operate. The gas transmission module 306 may include an airflow generator 3062 and a gas transmission pipe 3064 which are in communication with each other, so that the gas transmission state may be configured to control the opening and closing state of the gas generator 3062 and the opening and closing state of the gas transmission pipe 3064 according to the control parameter, to realize transporting gas or stop transporting gas.

In addition, the acquisition module 302 and the control module 304 may be connected by wired communication or wireless communication, and the control module 304 and the gas transmission module 306 may be connected by wired communication or wireless communication.

In this embodiment, the respiratory control assembly 30 is a servo control system that controls the gas transmission state of the gas transmission module 306 based on the gas parameter of the eustachian tube 202 of the user collected by the acquisition module 302. Particularly, the acquisition module 302 transmits the collected gas parameter of the eustachian tube 202 of the user to the control module 304. The control module 304 is capable to determine whether the eustachian tube 202 is under the opening state or under the closing state according to whether the gas parameter changes. If the eustachian tube 202 is under the opening state, the control module 304 generates a control instruction to control the gas transmission module 306 to stop transporting the gas, to eliminate the positive pressure in the eustachian tube 202, so that the eustachian tube 202 is automatically closed; if the eustachian tube 202 is under the closing state, the control module 304 generates a control instruction to control the gas transmission module 306 to continue transporting the gas, to make the respiratory control assembly 30 is capable to continuously transport the gas to a user for treatment.

By setting the respiratory control assembly 30 having the acquisition module 302, when a user is treated, the respiratory control assembly 30 is capable to improve the reliability of operation for a user with autonomous ability, and the treatment effect is further improved, and for a user without autonomous ability, this respiratory control assembly 30 is capable to prevent the user from generating risks of problems for example "ear perforation" and "hearing loss" during the treatment process, to improve the safety of operation.

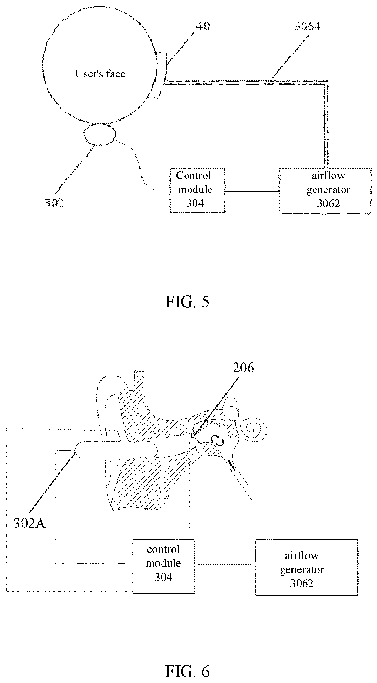

As shown in FIG. 6, in some embodiments, the acquisition module 302 includes an air pressure detector 302A and an in-ear structure for accommodating the air pressure detector 302A, among them, the gas parameter is an air pressure of an external auditory canal 208 connected with the eustachian tube 202, and the air pressure detector 302A is used for collecting the air pressure of the external auditory canal 208. The control module 304 is further configured for, when it is detected that the air pressure of the external auditory canal 208 is greater than an initial air pressure, determining that the eustachian tube 202 is open, and controlling the gas transmission module 306 to stop transporting gas, and when it is detected that the air pressure of the external auditory canal 208 drops to less than or equal to the initial air pressure, determining that the eustachian tube 202 is closed, and controlling the gas transmission module 306 to continue transporting the gas.

In this embodiment, as an implementation of the acquisition module 302, the air pressure detector 302A is used as the acquisition module 302, and the shape of the air pressure detector 302A is set as an in-ear probe structure. From the above description of the eustachian tube 202, it may be seen that the eustachian tube 202 is separated from the external auditory canal 208 by an ear-drum and correspondingly, the gas parameter is an air pressure of the external auditory canal 208. The in-ear air pressure detector 302A may contact with the skin of the external auditory canal 208 and close the external auditory canal 208, to collect the air pressure of the external auditory canal 208.

Particularly, if the eustachian tube 202 is open, when the eustachian tube 202 is open under the action of the gas transmission device, the airflow input by the gas transmission device enters a user's middle ear canal 204 through the eustachian tube 202, and the air pressure in the middle ear canal 204 gradually increases, so that the air pressure in the middle ear canal 204 is greater than the air pressure in the external ear canal 208, and the ear-drum 206 is compressed to cave into the external ear canal 208, as shown in FIG 6. Since the external auditory canal 208 is closed by the air pressure detector 302A, the volume of the external auditory canal 208 is reduced by the depression of the ear-drum, causing the air pressure in the external auditory canal 208 to increase thereafter, and the air pressure detector 302A feeds back to the detected air pressure of the external auditory canal 208 to the control module 304.

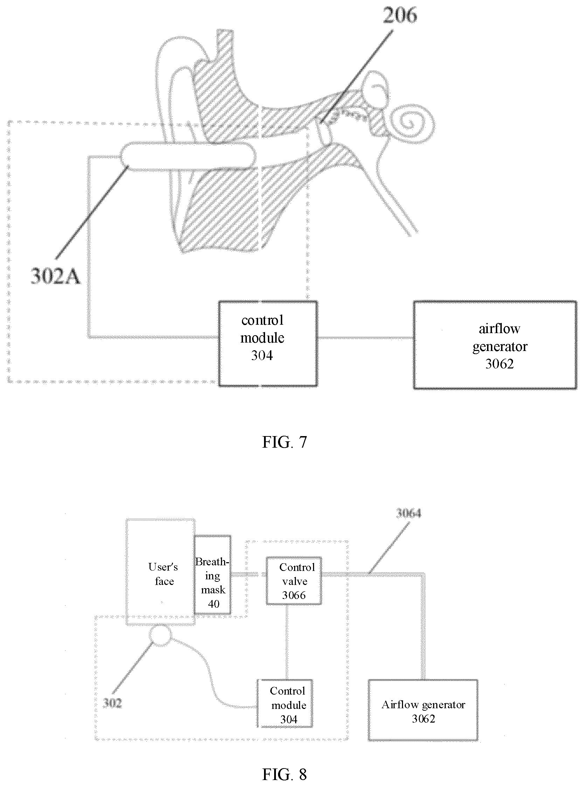

The control module 304 receives the air pressure parameter of the external auditory canal 208 from the acquisition module 302. If it is detected that the air pressure of the ear canal is increased relative to the initial air pressure, it indicates that the eustachian tube 202 has a risk of being open. At this time, the gas transmission module 306 is controlled to stop transporting gas, to eliminate the positive pressure of the middle auditory canal 204 and reset the eardrum. As shown in FIG. 7, the eustachian tube 202 is capable to be automatically closed within a short time after the positive pressure is eliminated, to prevent the user from being damaged the ear canal during the use of the respiratory control assembly 30, and the respiratory control assembly 30 is ensured continuously and reliably operating, to improve the safety of using the respiratory control assembly 30. In addition, the air pressure detector 302A is used as the acquisition module 302, and whether the eustachian tube 202 is open or not is determined based on the detected air pressure of the external auditory canal 208, so that the detection precision and accuracy are relatively high, and it is conducive to obtain the timely response of the gas transmission module 306.

After the eustachian tube 202 is closed, the air pressure detector 302A continuously collects the ear canal air pressure of the external auditory canal 208 and sends it to the control module 304. When it is detected that the ear canal air pressure returns to the initial air pressure, the control module 304 controls the gas transmission module 306 to resume transporting the gas, so that the respiratory control assembly 30 is capable to resume operation in time.

Optionally, the acquisition module 302 further includes an ear hanging structure, which is connected with the in-ear structure, and the ear hanging structure is used for being hung on an auricle of the user.

In this embodiment, by further disposing the ear hanging structure on the in-ear structure, the convenience of using the acquisition module 302 may be improved.

In addition, the number of the acquisition modules 302 is two, the two acquisition modules 302 are inserted into the left and right ears of a user during use, respectively. If the air pressure detected by any one of the two acquisition modules 302 rises from the initial air pressure or falls back to the initial air pressure, the control module 304 will generate a control instruction of the gas transmission module 306, so that the state of the gas transmission module 306 is suitable for the change of the air pressure in the external auditory canal 208.

In some embodiments, the acquisition module 302 includes an acoustic wave detector and a patch structure for accommodating the acoustic wave detector, wherein the acoustic wave detector is used for collecting acoustic wave information of breathing airflow. The control module 304 is used for, when it is detected that matching degree between a waveform of the acoustic wave information and a preset waveform is less than a preset matching threshold, determining that the eustachian tube 202 is open, and controlling the gas transmission module 306 to stop transporting gas, and when it is detected that the matching degree is greater than or equal to the preset matching threshold, determining that the eustachian tube 202 is closed, and controlling the gas transmission module 306 to continue transporting gas.

In this embodiment, as another implementation of the acquisition module 302, since the breathing airflow has an audio parameter, the acoustic wave detector may be used as the acquisition module 302 to determine whether the eustachian tube 202 is open by detecting the acoustic wave changes of a user's breathing airflow.

Particularly, the acoustic wave detector is designed to be in the form of a patch to be attached to a user's face, neck or ear root during use, to transmit an acoustic wave to the acoustic wave detector through the user's bones, flesh and blood and other media. When the eustachian tube 202 is closed, the breathing airflow merely passes through the nasal cavity tube and/or the oral cavity tube, and the acoustic wave of the breathing gas is transmitted to the acoustic wave detector through media for example the user's bones, flesh and blood, and the like. The waveform information of the acoustic wave collected by the acoustic wave detector when the eustachian tube 202 is closed is stored as a preset waveform, and when the eustachian tube 202 is open, the breathing airflow passes through the nasal cavity tube and/or the oral cavity tube and the eustachian tube 202, and the acoustic wave information of the breathing gas is transmitted to the acoustic wave detector through the user's bones, flesh and blood and other media.

The control module 304 compares the waveform of the received acoustic wave information with the preset waveform. Since a certain fluctuation generally exists in the acoustic wave waveform of a user's breathing airflow, when the waveform information of the breathing gas is collected in real time, by detecting the matching degree between the waveform information and the preset waveform, it is determined whether the user's breathing frequency parameter and the like fluctuate greatly based on the waveform of the acoustic wave information. Furthermore, when the significant fluctuation is detected, that is, the matching degree is less than the preset matching threshold, it is determined that the eustachian tube 202 is open; when it is detected that the matching degree is less than a preset matching threshold, it is determined that the eustachian tube 202 is open, and when it is detected that the matching degree is greater than or equal to the preset matching threshold, it is determined that the eustachian tube 202 is closed.

When the eustachian tube 202 is open, the gas transmission module 306 is controlled to stop transporting the gas. When the eustachian tube 202 is closed, the gas transmission module 306 is controlled to continue transporting the gas. In the manner of using the acoustic wave detector as the acquisition module 302, the acoustic wave detector provided as the patch structure may be attached to a user's face, neck or ear root and other parts by using sticky substances, so that the acquisition mode has high convenience while achieving the detection function of opening and closing states of the eustachian tube 202.

Among them, the waveform information may particularly include data for example an amplitude, a frequency and a period of the acoustic wave.

In some embodiment, the respiratory control assembly 30 particularly includes an acquisition module 302, a control module 304, a gas transmission module 306 and a prompting module, among them, the acquisition module 302 is connected with the control module 304 in communication, the control module 304 is connected with the gas transmission module 306 in communication, and the prompting module is connected with the control module 304 in communication. The prompting module is used for sending prompting information when it is determined by the control module 304 that the eustachian tube 202 is under a working condition of being open.

In this embodiment, by adding the prompting module, the prompting information may be generated while the gas transmission state of the gas transmission module 306 is adaptively configured, so that other personnel may be prompted that the current user has the risk of opening the eustachian tube 202, which is beneficial to further reducing the risk that the eustachian tube 202 of the user is continuously under the opening state, to further improve the use safety of the respiratory control assembly 30.

Particularly, the prompting module may be at least one of a buzzer, a speaker, a display screen and a communication device.

As shown in FIG. 5, in some embodiments, the respiratory control assembly 30 particularly includes an acquisition module 302, a control module 304 and a gas transmission module 306 which are sequentially connected in communication. Among them, the gas transmission module 306 includes an airflow generator 3062 and a controlled device which are connected in communication, and a gas transmission pipe 3064 which is connected with the airflow generator 3062. The controlled device and the control module 304 are connected in communication, and the controlled device receives a control parameter of the control module, to control the airflow generator 3062 to transport gas through the gas transmission pipe 3064 or stop transporting gas according to the control parameter.

Particularly, the acquisition module 302 may include an air pressure detector 302A and an in-ear structure for accommodating the air pressure detector 302A, among them, the gas parameter is an air pressure of the external auditory canal 208 connected with the eustachian tube 202, and the air pressure detector 302A is used for collecting the air pressure of the external auditory canal 208.

Alternatively, the acquisition module 302 may include an acoustic wave detector and a patch structure for accommodating the acoustic wave detector, and the acoustic wave detector is used for collecting the acoustic information of the breathing airflow.

It may be understood by a person skilled in the art that, the controlled device may be an internal device of the airflow generator 3062 or an external device of the airflow generator 3062. The controlled device controls the airflow generator 3062 to transport gas through the gas transmission pipe 3064 or stop transporting gas by receiving the control parameter sent by the control module 304, particularly by controlling the airflow generator 3062 to run or stop running, or controlling the gas transmission pipe 3064 to be communicated or to be cut-off, to achieve the purpose of closing the eustachian tube 202 by quickly eliminating the positive pressure.

In some embodiments, the controlled device is a switch controller of the airflow generator 3062, one end of the gas transmission pipe 3064 is communicated with the airflow generator 3062, and the other end of the gas transmission pipe 3064 is configured for transporting gas to the user. The control parameter is used for controlling the switch controller to turn off the airflow generator 3062 when the eustachian tube 202 is under the working condition of being open, and the control parameter is used for controlling the switch controller to turn on the airflow generator 3062 when the eustachian tube 202 is under the working condition of being closed.

In this embodiment, as a simple control method, the switch controller of the airflow generator 3062 is used as a controlled device, to make the airflow generator 3062 being directly controlled by the control module 304, so that the gas transmission state may be directly and reliably controlled.

In some embodiments, the controlled device is a control valve 3066, the control valve 3066 is used for controlling the gas transmission pipe 3064 to be communicated or to be cut-off, and the control parameter is used for controlling the control valve 3066 to be closed when the eustachian tube 202 is under the working condition of being open, and the control parameter is used to control the control valve 3066 to be open when the eustachian tube 202 is under the working condition of being closed.

In this embodiment, as another simple control method, the control valve 3066 is disposed on the gas transmission pipe 3064 and used as a controlled device. When the eustachian tube 202 is detected to be open, merely the gas transmission pipe 3064 is controlled to be cut-off to stop transporting gas. At this time, the airflow generator 3062 may still under an operation state, and when the eustachian tube 202 is determined to be closed, the gas transmission pipe 3064 is controlled to be communicated, so that the airflow may be transported to a user in time. In this way, the influence of stopping transporting gas on users may be reduced as much as possible, and the safety and reliability of the respiratory control assembly 30 during use are further ensured.

Referring to FIG. 8, it shows a structural block diagram of an embodiment of the ventilation therapy apparatus in the present application, the ventilation therapy apparatus may particularly include the following modules: a breathing mask 40; the respiratory control assembly 30 according to any one of the above-mentioned embodiments, the respiratory control assembly 30 includes an gas transmission pipe 3064 and an airflow generator 3062, one end of the gas transmission pipe 3064 is connected to the breathing mask 40, and the other end of the gas transmission pipe 3064 is connected to the airflow generator 3062.

Particularly, the ventilation therapy apparatus includes a breathing mask 40, an acquisition module 302, a control module 304 and a gas transmission module 306, among them, the acquisition module 302 may include an air pressure detector 302A and/or an acoustic wave detector. For the air pressure detector, the air pressure detector 302A is provided as an in-ear structure, the gas parameter is an air pressure of an external auditory canal 208 connected with the eustachian tube 202, and the air pressure detector 302A is used for collecting the air pressure of the external auditory canal 208. The control module 304 is further configured for, when it is detected that the air pressure of the external auditory canal 208 is greater than an initial air pressure, determining that the eustachian tube is open, and controlling the gas transmission module 306 to stop transporting gas, and when it is detected that the air pressure of the external auditory canal 208 drops to be less than or equal to the initial air pressure, determining that the eustachian tube 202 is closed, and controlling the gas transmission module 306 to continue transporting gas. For the acoustic wave detector, the acoustic wave detector is provided as a patch structure. The acoustic wave detector is used for collecting acoustic wave information of breathing airflow. The control module 304 is used for, when it is detected that matching degree between a waveform of the acoustic wave information and a preset waveform is less than a preset matching threshold, determining that the eustachian tube 202 is open, and controlling the gas transmission module 306 to stop transporting gas, and when it is detected that the matching degree is greater than or equal to the preset matching threshold, determining that the eustachian tube 202 is closed, and controlling the gas transmission module 306 to continue transporting gas.

Among them, the breathing mask 40 may be any one of a nasal mask covering merely the nose, an oral mask covering merely the mouth, an oral and nasal mask covering both the mouth and the nose (also called a full-face mask) or a nasal pad mask inserted into the nostril.

In this embodiment, the collected gas parameter of a user's eustachian tube 202 is transmitted to the control module 304 through the acquisition module 302, and the control module 304 is capable to determine whether the eustachian tube 202 is under the opening state or the closing state according to whether the gas parameter changes. If the eustachian tube 202 is under the opening state, the control module 304 generates a control instruction to control the gas transmission module 306 to stop transporting gas, to eliminate the positive pressure in the eustachian tube 202. Further, the eustachian tube 202 is automatically closed. If the eustachian tube 202 is under the closing state, the control module 304 generates a control instruction to control the gas transmission module 306 to continue transporting gas, so that the ventilation therapy apparatus is capable to continuously transport gas to a user for treatment.

By providing the ventilation therapy apparatus having the acquisition module 302, when a user is treated, the ventilation therapy apparatus is capable to improve the reliability of operation for a user with autonomous ability, and the treatment effect is effectively improved, and for a user without autonomous ability, the ventilation therapy apparatus is capable to prevent the user from risks of generating problems for example "ear perforation" and hearing loss and the like during treatment, to improve the safety of operation.

Optionally, the control module 304 may directly control the opening and closing of the airflow generator 3062, to control the gas transmission module 306 to transport gas or stop transporting gas.

Optionally, under the condition that the controlled device is a control valve 3066, the control valve 3066 is disposed on the gas transmission pipe 3064, and/or the control valve 3066 is disposed at a joint of the gas transmission pipe 3064 and the breathing mask 40, and/or the control valve 3066 is disposed at a joint of the gas transmission pipe 3064 and the airflow generator 3062.

Particularly, the control valve 3066 is essentially a switch that may control the gas transmission pipe 3064 to be communicated or to be cut-off. The control valve 3066 may be installed on the gas transmission pipe 3064, or at the joint of the breathing mask 40 and the gas transmission pipe 3064, or the control valve 3066 is disposed at the joint of the gas transmission pipe 3064 and the airflow generator 3062. The opening and closing of the control valve 3066 are controlled by the control module 304. When the control module 304 sends a "close" instruction, the control valve 3066 is closed, so that the gas transmission pipe 3064 is cut-off, and the airflow from the airflow generator 3062 cannot enter a user's airway. When the control module 304 sends an "open" instruction, the control valve 3066 is open, and the gas is transported normally.

Every embodiment in the present description is described in a progressive way, and every embodiment focuses on the differences from other embodiments, so it is merely necessary to refer to the same and similar portions of every embodiment.

Finally, it should also be noted that in this paper, relational terms for example first and second are merely used to distinguish one entity or operation from another entity or operation, and do not necessarily require or imply any such actual relationship or order between these entities or operations. Moreover, the terms "comprise", "include" or any other variation thereof are intended to cover non-exclusive inclusion, so that a process, a method, a product or a terminal apparatus including a series of elements includes not only those elements, but also other elements not explicitly listed, or elements inherent to such process, method, product or terminal apparatus. Without more restrictions, the element defined by the phrase "including one" does not exclude that other identical elements exist in the process, method, product or terminal apparatus including the element.

In the present application, the terms "first", "second" and "third" are merely used for descriptive purposes and cannot be understood as indicating or implying relative importance; The term "a plurality of" refers to two or more, unless otherwise explicitly defined. Terms for example "installation", "connect", "communicate" and "fix" should be understood broadly. For example, "connect" may be a fixed connection, a detachable connection or an integral connection; "communicate" may be directly communicated or indirectly communicated through an intermediary medium. For a person skilled in the art, the specific meanings of the above-mentioned terms in the present application may be understood according to the specific circumstances.

In the description of the present application, it should be understood that, the orientation or position relationship indicated by the terms "upper", "lower", "left", "right", "front" and "rear" is based on the orientation or position relationship shown in the drawings, which is merely for the convenience of describing the present application and simplifying the description, and does not indicate or imply that the referred device or unit must have a specific direction, be constructed and operated in a specific orientation.

In the description of the present specification, descriptions of the terms "one embodiment", "some embodiments" and "specific embodiments" mean that specific features, structures, materials or characteristics described in connection with this embodiment or example are included in at least one embodiment or example of this application. In the present specification, the schematic expressions of the above-mentioned terms do not necessarily refer to the same embodiment or example. Moreover, the specific features, structures, materials or characteristics described may be combined in any one or more embodiments or examples in a suitable manner.

## Claims

1. A respiratory control assembly (30) comprising
an acquisition module (302), a control module (304) and a gas transmission module (306), wherein,
the acquisition module (302) is connected with the control module (304) in communication, and is used for collecting a gas parameter of an eustachian tube (202) of a user and sending the gas parameter to the control module (304);
the control module (304) is connected with the gas transmission module (306) in communication, and
the control module (304) is used for determining opening and closing states of the eustachian tube (202) according to the gas parameter, and configuring a gas transmission state of the gas transmission module (306) according to the opening and closing states.

2. The respiratory control assembly (30) according to claim 1, wherein
the acquisition module (302) comprises an air pressure detector (302A) and an in-ear structure for accommodating the air pressure detector (302A), wherein the gas parameter is an air pressure of an external auditory canal (208) connected with the eustachian tube (202), and the air pressure detector (302A) is used for collecting the air pressure of the external auditory canal (208); and
the control module (304) is further configured for, when it is detected that the air pressure of the external auditory canal (208) is greater than an initial air pressure, determining that the eustachian tube (202) is open, and controlling the gas transmission module (306) to stop transporting gas, and when it is detected that the air pressure of the external auditory canal (208) drops to less than or equal to the initial air pressure, determining that the eustachian tube (202) is closed, and controlling the gas transmission module (306) to continue transporting the gas.

3. The respiratory control assembly (30) according to claim 2, wherein the acquisition module (302) further comprises:
an ear hanging structure connected with the in-ear structure, wherein the ear hanging structure is used for being hung on an auricle of the user.

4. The respiratory control assembly (30) according to claim 1, wherein
the acquisition module (302) comprises an acoustic wave detector and a patch structure for accommodating the acoustic wave detector, wherein the acoustic wave detector is used for collecting acoustic wave information of breathing airflow; and
the control module (304) is used for, when it is detected that matching degree between a waveform of the acoustic wave information and a preset waveform is less than a preset matching threshold, determining that the eustachian tube (202) is open, and controlling the gas transmission module (306) to stop transporting the gas, and when it is detected that the matching degree is greater than or equal to the preset matching threshold, determining that the eustachian tube (202) is closed, and controlling the gas transmission module (306) to continue transporting the gas.

5. The respiratory control assembly (30) according to any one of claims 1-4, wherein the respiratory control assembly (30) further comprises:
a prompting module connected with the control module (304) in communication, wherein the prompting module is used for sending prompting information when it is determined by the control module (304) that the eustachian tube (202) is under a working condition of being open.

6. The respiratory control assembly (30) according to any one of claims 1-4, wherein the gas transmission module (306) comprises an airflow generator (3062) and a controlled device which are connected in communication, and a gas transmission pipe (3064) which is connected with the airflow generator (3062); the controlled device and the control module (304) are connected in communication, and the controlled device receives a control parameter of the control module (304), to control the airflow generator (3062) to transport gas through the gas transmission pipe (3064) or stop transporting gas according to the control parameter.

7. The respiratory control assembly (30) according to claim 6, wherein the controlled device is a switch controller of the airflow generator (3062), one end of the gas transmission pipe (3064) is communicated with the airflow generator (3062), and the other end of the gas transmission pipe (3064) is used for transporting the gas to the user; the control parameter is used for controlling the switch controller to turn off the airflow generator (3062) when the eustachian tube (202) is under the working condition of being open, and the control parameter is used for controlling the switch controller to turn on when the eustachian tube (202) is under the working condition of being closed.

8. The respiratory control assembly (30) according to claim 6, wherein the controlled device is a control valve (3066), the control valve (3066) is used for controlling the gas transmission pipe (3064) to be communicated or to be cut-off, and the control parameter is used for controlling the control valve (3066) to be closed when the eustachian tube (202) is under the working condition of being open, and the control parameter is used to control the control valve (3066) to be opened when the eustachian tube (202) is under the working condition of being closed.

9. A ventilation therapy apparatus comprising:
a breathing mask (40); and
the respiratory control assembly (30) according to any one of claims 1 to 8, wherein the respiratory control assembly (30) comprises a gas transmission pipe (3064) and an airflow generator (3062), wherein one end of the gas transmission pipe (3064) is connected to the breathing mask (40), and the other end of the gas transmission pipe (3064) is connected to the airflow generator (3062).

10. The ventilation therapy apparatus according to claim 9, wherein the respiratory control assembly (30) comprises a controlled device;
under the condition that the controlled device is a control valve (3066), the control valve (3066) is disposed on the gas transmission pipe (3064), and/or the control valve (3066) is disposed at a joint of the gas transmission pipe (3064) and the breathing mask (40), and/or the control valve (3066) is disposed at a joint of the gas transmission pipe (3064) and the airflow generator (3062).

## Patentansprüche

1. Eine Atemkontrollvorrichtung (30), aufweisend
ein Erfassungsmodul (302), ein Steuerungsmodul (304) und ein Gasübertragungsmodul (306), wobei
das Erfassungsmodul (302) mit dem Steuerungsmodul (304) in Verbindung steht und zum Erfassen eines Gasparameters einer Eustachischen Röhre (202) eines Benutzers und zum Senden des Gasparameters an das Steuerungsmodul (304) verwendet wird;
das Steuerungsmodul (304) mit dem Gasübertragungsmodul (306) in Verbindung steht und
das Steuerungsmodul (304) dazu dient, Öffnung und Schließzustand
der Eustachischen Röhre (202) anhand des Gasparameters zu bestimmen und einen Gasübertragungszustand des Gasübertragungsmoduls (306) entsprechend den Öffnung und Schließzuständen zu konfigurieren.

2. Die Atemkontrollvorrichtung (30) gemäß Anspruch 1, wobei
das Erfassungsmodul (302) einen Luftdruckdetektor (302A) und eine In-Ear-Struktur zur Aufnahme des Luftdruckdetektors (302A) umfasst, wobei der Gasparameter ein Luftdruck eines mit der Eustachischen Röhre (202) verbundenen äußeren Gehörgangs (208) ist, und der Luftdruckdetektor (302A) zum Erfassen des Luftdrucks des äußeren Gehörgangs (208) verwendet wird; und
das Steuerungsmodul (304) weiter so konfiguriert ist, dass es, wenn festgestellt wird, dass der Luftdruck des äußeren Gehörgangs (208) größer als ein Anfangsluftdruck ist, bestimmt, dass die Eustachische Röhre (202) offen ist, und das Gasübertragungsmodul (306) so zu steuern, dass es den Transport von Gas stoppt, und wenn festgestellt wird, dass der Luftdruck des äußeren Gehörgangs (208) auf weniger als oder gleich den Anfangsluftdruck abfällt, als Bestimmungsvorrichtung anzugeben, dass die Eustachische Röhre (202) geschlossen ist, und das Gasübertragungsmodul (306) so zu steuern, dass es den Transport des Gases fortsetzt.

3. Die Atemkontrollvorrichtung (30) gemäß Anspruch 2, wobei das Erfassungsmodul (302) weiter aufweist:
eine mit der In-Ear-Struktur verbundene Ohrhängestruktur, wobei die Ohrhängestruktur dazu dient, an einer Ohrmuschel des Benutzers aufgehängt zu werden.

4. Die Atemkontrollvorrichtung (30) gemäß Anspruch 1, wobei
das Erfassungsmodul (302) einen Schallwellendetektor und eine Patch-Struktur zur Aufnahme des Schallwellendetektors aufweist, wobei der Schallwellendetektor zum Sammeln von Schallwelleninformationen des Atemluftstroms verwendet wird; und
das Steuerungsmodul (304) dazu dient, wenn festgestellt wird, dass der Übereinstimmungsgrad zwischen einer Wellenform der Schallwelleninformationen und einer voreingestellten Wellenform unter einem voreingestellten Übereinstimmungsschwellenwert liegt, zu bestimmen, dass die Eustachische Röhre (202) offen ist, und das Gasübertragungsmodul
(306) so zu steuern, dass der Transport des Gases gestoppt wird, und wenn festgestellt wird, dass der Übereinstimmungsgrad größer oder gleich dem voreingestellten Übereinstimmungsschwellenwert ist, zu bestimmen, dass die Eustachische Röhre (202) geschlossen ist, und das Gasübertragungsmodul (306) so zu steuern, dass der Transport des Gases fortgesetzt wird.

5. Die Atemkontrollvorrichtung (30) gemäß einem der Ansprüche 1 bis 4, wobei
die Atemkontrollvorrichtung (30) weiter aufweist:
ein Aufforderungsmodul, das mit dem Steuerungsmodul (304) in Verbindung steht, wobei das Aufforderungsmodul zum Senden von Aufforderungsinformationen verwendet wird, wenn vom Steuerungsmodul (304) festgestellt wird, dass sich die Eustachische Röhre (202) in einem offenen Betriebszustand befindet.

6. Die Atemkontrollvorrichtung (30) gemäß einem der Ansprüche 1 bis 4, wobei das Gasübertragungsmodul (306) einen Luftstromgenerator (3062) und eine gesteuerte Vorrichtung aufweist, die miteinander verbunden sind, sowie eine Gasübertragungsleitung (3064), die mit dem Luftstromgenerator (3062) verbunden ist; die gesteuerte Vorrichtung und das Steuerungsmodul (304) miteinander verbunden sind und die gesteuerte Vorrichtung einen Steuerparameter des Steuerungsmoduls (304) empfängt, um die Luftstromgenerator (3062) so zu steuern, dass er Gas durch die Gasübertragungsleitung (3064) transportiert oder den Gastransport gemäß dem Steuerparameter stoppt.

7. Die Atemkontrollvorrichtung (30) gemäß Anspruch 6, wobei
die gesteuerte Vorrichtung eine Steuereinheit des Luftstromgenerators (3062) ist, ein Ende der Gasübertragungsleitung (3064) mit dem Luftstromgenerator (3062) in Verbindung steht und das andere Ende der Gasübertragungsleitung (3064) zum Transportieren des Gases zum Benutzer verwendet wird; der Steuerparameter wird dazu verwendet, die Steuereinheit so zu steuern, dass der Luftstromgenerator (3062) ausgeschaltet wird, wenn sich die Eustachische Röhre (202) in der Öffnung befindet, und der Steuerparameter wird dazu verwendet, die Steuereinheit so zu steuern, dass sie eingeschaltet wird, wenn sich die Eustachische Röhre (202) im geschlossenen Betriebszustand befindet.

8. Die Atemkontrollvorrichtung (30) gemäß Anspruch 6, wobei
die gesteuerte Vorrichtung ein Steuerventil (3066) ist, das Steuerventil (3066) dazu dient, die Gasübertragungsleitung (3064) zu verbinden oder zu unterbrechen, und der Steuerparameter dazu dient, das Steuerventil (3066) zu schließen, wenn sich die Eustachische Röhre (202) im geöffneten Betriebszustand ist, und der Steuerparameter dazu verwendet wird, das Steuerventil (3066) zu öffnen, wenn sich die Eustachische Röhre (202) im geschlossenen Betriebszustand befindet.

9. Eine Beatmungstherapieeinrichtung, aufweisend:
eine Atemmaske (40); und
die Atemkontrollvorrichtung (30) gemäß einem der Ansprüche 1 bis 8, wobei die Atemkontrollvorrichtung (30) eine Gasübertragungsleitung (3064) und einen Luftstromgenerator (3062) aufweist, wobei ein Ende der Gasübertragungsleitung (3064) mit der Atemmaske (40), verbunden ist und das andere Ende des Gasübertragungsrohrs (3064) mit dem Luftstromgenerator (3062) verbunden ist.

10. Die Beatmungstherapieeinrichtung gemäß Anspruch 9, wobei die Atemkontrollvorrichtung (30) eine gesteuerte Vorrichtung aufweist;
unter der Bedingung, dass die gesteuerte Vorrichtung ein Steuerventil (3066) ist, das Steuerventil (3066) an der Gasübertragungsleitung (3064) angeordnet ist und/oder das Steuerventil (3066) an einer Verbindung der Gasübertragungsleitung (3064) und der Atemmaske (40) angeordnet ist und/oder das Steuerventil (3066) an einer Verbindung der Gasübertragungsleitung (3064) und des Luftstromgenerators (3062) angeordnet ist.

## Revendications

1. Ensemble de contrôle respiratoire (30) comprenant
un module d'acquisition (302), un module de commande (304) et un module de transmission de gaz (306), dans lequel,
le module d'acquisition (302) est raccordé au module de commande (304) en communication, et est utilisé pour collecter un paramètre de gaz d'une trompe d'Eustache (202) d'un utilisateur et pour envoyer le paramètre de gaz au module de commande (304) ;
le module de commande (304) est raccordé au module de transmission de gaz (306) en communication, et
le module de commande (304) est utilisé pour déterminer des états d'ouverture et de fermeture de la trompe d'Eustache (202) en fonction du paramètre de gaz, et pour configurer un état de transmission du gaz du module de transmission de gaz (306) en fonction des états d'ouverture et de fermeture.

2. Ensemble de contrôle respiratoire (30) selon la revendication 1, dans lequel
le module d'acquisition (302) comprend un détecteur de pression d'air (302A) et une structure intra-auriculaire pour loger le détecteur de pression d'air (302A), dans lequel le paramètre de gaz est une pression d'air d'un canal auditif externe (208) raccordé à la trompe d'Eustache (202), et le détecteur de pression d'air (302A) est utilisé pour collecter la pression d'air du canal auditif externe (208); et
le module de commande (304) est en outre configuré pour, lorsqu'il est détecté que la pression d'air du canal auditif externe (208) est supérieure à une pression d'air initiale, déterminer que la trompe d'Eustache (202) est ouverte, et pour commander au module de transmission de gaz (306) de stopper le transport de gaz, et lorsqu'il est détecté que la pression d'air du canal auditif externe (208) chute en dessous ou au même niveau que la pression d'air initiale, déterminer que la trompe d'Eustache (202) est fermée, et pour commander au module de transmission de gaz (306) de continuer à transporter le gaz.

3. Ensemble de contrôle respiratoire (30) selon la revendication 2, dans lequel
le module d'acquisition (302) comprend en outre :
une structure suspendue à l'oreille raccordée à la structure intra-auriculaire, dans laquelle la structure suspendue à l'oreille est utilisée pour être suspendue à une oreille de l'utilisateur.

4. Ensemble de contrôle respiratoire (30) selon la revendication 1, dans lequel
le module d'acquisition (302) comprend un détecteur d'onde acoustique et une structure type timbre pour loger le détecteur d'onde acoustique, dans lequel le détecteur d'onde acoustique est utilisé pour collecter des informations d'onde acoustique du flux d'air respiratoire ; et
le module de commande (304) est utilisé pour, lorsqu'il est détecté qu'un degré de correspondance entre une forme d'onde des informations d'onde acoustique et une forme d'onde prédéfinie est inférieur à un seuil de correspondance prédéfini, déterminer que la trompe d'Eustache (202) est ouverte, et commander au module de transmission de gaz (306) de stopper le transport du gaz, et lorsqu'il est détecté que le degré de correspondance est supérieur ou égal au seuil de correspondance prédéfini, déterminer que la trompe d'Eustache (202) est fermée, et commander au module de transmission de gaz (306) de continuer à transporter le gaz.

5. Ensemble de contrôle respiratoire (30) selon l'une quelconque des revendications 1 à 4, dans lequel
l'ensemble de contrôle respiratoire (30) comprend en outre :
un module d'alerte raccordé au module de commande (304) en communication, dans lequel le module d'alerte est utilisé pour envoyer des informations d'alerte lorsqu'il est déterminé par le module de commande (304) que la trompe d'Eustache (202) se trouve sous un état de travail dans lequel elle est ouverte.

6. Ensemble de contrôle respiratoire (30) selon l'une quelconque des revendications 1 à 4, dans lequel
le module de transmission de gaz (306) comprend un générateur de flux d'air (3062) et un dispositif contrôlé qui sont raccordés en communication, et un tuyau de transmission de gaz (3064) qui est raccordé au générateur de flux d'air (3062) ; le dispositif contrôlé et le module de commande (304) sont raccordés en communication, et le dispositif contrôlé reçoit un paramètre de commande du module de commande (304), pour commander au générateur de flux d'air (3062) de transporter du gaz à travers le tuyau de transmission de gaz (3064) ou d'arrêter le transport de gaz en fonction du paramètre de commande.

7. Ensemble de contrôle respiratoire (30) selon la revendication 6, dans lequel
le dispositif contrôlé est un contrôleur de commutation du générateur de flux d'air (3062), une extrémité du tuyau de transmission de gaz (3064) communique avec le générateur de flux d'air (3062), et l'autre extrémité du tuyau de transmission de gaz (3064) est utilisée pour transporter le gaz vers l'utilisateur ; le paramètre de commande est utilisé pour commander au contrôleur de commutation d'arrêter le générateur de flux d'air (3062) lorsque la trompe d'Eustache (202) se trouve sous l'état de travail d'être ouverte, et le paramètre de commande est utilisé pour commander la mise en marche au contrôleur de commutation lorsque la trompe d'Eustache (202) se trouve sous l'état de travail dans lequel elle est fermée.

8. Ensemble de contrôle respiratoire (30) selon la revendication 6, dans lequel
le dispositif contrôlé est une soupape de régulation (3066), la soupape de régulation (3066) est utilisée pour commander la mise en communication ou le blocage de communication du tuyau de transmission de gaz (3064), et le paramètre de commande est utilisé pour commander à la soupape de régulation (3066) de se fermer lorsque la trompe d'Eustache (202) se trouve sous l'état de travail dans lequel elle est ouverte, et le paramètre de commande est utilisé pour commander l'ouverture de la soupape de régulation (3066) lorsque la trompe d'Eustache (202) se trouve sous l'état de travail d'être fermée.

9. Appareil de thérapie par ventilation comprenant :
un masque respiratoire (40) ; et
l'ensemble de contrôle respiratoire (30) selon l'une quelconque des revendications 1 à 8, dans lequel l'ensemble de contrôle respiratoire (30) comprend un tuyau de transmission de gaz (3064) et un générateur de flux d'air (3062), dans lequel une extrémité du tuyau de transmission de gaz (3064) est raccordée au masque respiratoire (40), et l'autre extrémité du tuyau de transmission de gaz (3064) est raccordée au générateur de flux d'air (3062).

10. Appareil de thérapie par ventilation selon la revendication 9, dans lequel
l'ensemble de contrôle respiratoire (30) comprend un dispositif contrôlé ;
sous la condition que le dispositif contrôlé est une soupape de régulation (3066), la soupape de régulation (3066) est disposée sur le tuyau de transmission de gaz (3064), et/ou la soupape de régulation (3066) est disposée au niveau d'une jonction entre le tuyau de transmission de gaz (3064) et le masque respiratoire (40), et/ou la soupape de régulation (3066) est disposée au niveau d'une jonction entre le tuyau de transmission de gaz (3064) et le générateur de flux d'air (3062).
